# FASCICULE DE BREVET EUROPEEN

(11) **EP 4 035 177 B1**
(45) Date de publication et mention de la délivrance du brevet: **09.07.2025**
(21) Numéro de dépôt: 20790362.6
(22) Date de dépôt: 22.09.2020
(51) Int. Cl.: G16H 40/63, G06F 1/3234, G06F 1/3215, G06F 1/3287, G06F 1/16, G06F 3/0488, G16H 20/70, G16H 20/60, G16H 20/40

(54) **SYSTEME ET METHODE DE COMMANDE D'UN DISPOSITIF MEDICAL AGENCE POUR ETRE IMPLANTE**
SYSTEM UND VERFAHREN ZUR STEUERUNG EINER ZUR IMPLEMENTIERUNG ANGEORDNETEN MEDIZINISCHEN VORRICHTUNG
SYSTEM AND METHOD FOR CONTROLLING A MEDICAL DEVICE ARRANGED TO BE IMPLEMENTED

(30) Priorité: 23.09.2019 FR 1910454
(43) Date de publication de la demande: 03.08.2022
(73) Titulaire: Uromems, 38320 Eybens (FR)
(72) Inventeur: LAMRAOUI, Hamid, 38410 Vaulnaveys le Haut (FR); MARIEN, Marc, 38700 La Tronche (FR)
(74) Mandataire: Regimbeau
(86) Numéro de dépôt international: PCT/FR2020/051652
(87) Numéro de publication internationale: WO 2021/058906

(56) Documents cités:
- WO-A1-2010/007574
- US-A1- 2018 193 130
- HACHED SAMI ET AL: "Novel, Remotely Controlled, Artificial Urinary Sphincter: A Retro-Compatible Device", IEEE / ASME TRANSACTIONS ON MECHATRONICS, IEEE SERVICE CENTER, PISCATAWAY, NJ, US, vol. 19, no. 4, 25 April 2014 (2014-04-25), pages 1352 - 1362, XP011546547, ISSN: 1083-4435, [retrieved on 20140428], DOI: 10.1109/TMECH.2013.2280575

## Description

### DOMAINE DE L'INVENTION

La présente invention concerne un système de commande d'un dispositif médical implantable dans un corps humain ou animal.

L'invention concerne en particulier les dispositifs médicaux implantables actifs, c'est-à-dire les dispositifs médicaux dépendant pour leur fonctionnement d'une source d'énergie électrique et pour lesquels il est nécessaire de disposer d'un système de commande adapté.

### ARRIERE PLAN DE L'INVENTION

Il existe de nombreux dispositifs médicaux implantables dans un patient pour remplir une fonction dans le corps du patient, par exemple pour pallier un dysfonctionnement d'un organe naturel.

En particulier, on connait des dispositifs médicaux implantables destinés à obturer sélectivement un conduit anatomique, par exemple pour remédier à une incontinence (cas des sphincters urinaires et anaux artificiels) ou pour limiter l'entrée d'aliments dans l'estomac (cas des bandes gastriques). L'obturation du conduit anatomique est généralement engendrée par la compression exercée par une manchette enroulée autour dudit conduit.

D'une manière générale, un sphincter urinaire artificiel comprend une manchette occlusive destinée à être implantée autour de l'urètre, éventuellement du col vésical lors d'une implantation chez la femme ou de la prostate chez l'homme, de sorte à comprimer l'urètre pour éviter des fuites urinaires, notamment dans le cas où le patient souffre d'incontinence. Il est aussi prévu un système de commande agencé pour activer une commande du sphincter artificiel, par exemple un ajustement de la compression exercée par la manchette de sorte à obturer ou ouvrir le conduit naturel.

Dans les sphincters artificiels les plus anciens, le dispositif médical est agencé pour qu'une action manuelle du patient, par exemple une pression exercée sur un dispositif de pompe agencé sous la peau, active la commande du sphincter artificiel.

De nos jours, des systèmes plus perfectionnés sont en cours de développement afin d'éviter au patient une pression manuelle sur la pompe pour contrôler la manchette.

Le sphincter artificiel comprend alors un système de commande à distance, non implanté dans le corps du patient, et qui est adapté pour activer une commande du dispositif médical implanté, par exemple en réalisant une diminution de la pression exercée par la manchette sur le conduit naturel.

Il existe actuellement différents types de dispositifs médicaux implantés activables à distance par un système de commande non-implanté dans le corps du patient, par exemple des sphincters urinaires artificiels, mettant en oeuvre différentes technologies de manchettes (hydraulique, mécanique, gonflable etc.) et différentes technologies d'actionneurs piézo-électriques, des câbles etc.

Il est entendu que l'invention n'est pas limitée à une technologie particulière de dispositif médical implanté.

Toutefois, les dispositifs médicaux implantés activables à distance par un système de commande non-implanté dans le corps du patient présentent le désavantage de pouvoir être activés à tort, c'est-à-dire de manière non-volontaire par le patient. C'est généralement le cas lors d'une manipulation accidentelle du système de commande, par exemple lorsque le système de commande se présente sous la forme d'une télécommande avec un bouton déclenchant l'activation d'une commande du dispositif médical implanté et que cette télécommande est placée dans une poche du pantalon du patient. Dans ce cas, une simple modification de la posture du patient peut entrainer une pression involontaire sur le bouton de la télécommande et donc une activation d'une commande du dispositif médical, par exemple la réduction de la pression dans la manchette et la miction involontaire du patient.

Ces conditions occasionnent des situations très difficiles à supporter pour les patients. Il existe donc un besoin d'un système de commande à distance d'un dispositif médical implanté dans lequel on évite les commandes involontaires.

L'article de Sami Hached et al intitulé « Novel, Remotely Controlled, Artificial Urinary Sphincter : A Retro-Compatible Device », IEEE / ASME Transactions on Mechatronics, vol. 19, no. 4, 25 avril 2014, pp 1352-1362 divulgue un système de commande à distance d'un sphincter urinaire artificiel implantable au moyen d'un smartphone. Le document US 2018/0193130 divulgue un système de commande d'un dispositif médical implanté par action mécanique dans ou sur le corps du patient. Le document WO 2010/007574 décrit un dispositif de délivrance d'un médicament sécurisé comprenant un système de verrouillage qui ne peut être désactivé que par pression simultanée sur deux boutons et/ou par reconnaissance d'empreintes digitales

### EXPOSE DE L'INVENTION

La présente invention a donc pour but de remédier à ces inconvénients en proposant un système de commande à distance d'un dispositif médical implanté dans un corps humain ou animal qui active une commande seulement à la suite d'une action volontaire du patient.

A cet effet la présente invention propose un système de commande à distance d'un dispositif médical agencé pour être implanté dans un corps humain ou animal, selon la revendication 1.

L'invention concerne donc un système de commande non-implanté dans le corps du patient apte à commander à distance un dispositif médical qui lui est implanté dans le corps humain ou animal. Par implantation, on comprend qu'il s'agit d'un dispositif médical qui est introduit dans le corps humain ou animal, par exemple lors d'une opération chirurgicale.

Avantageusement, on notera que l'activation d'une commande du dispositif médical implanté par le déclenchement de deux éléments d'activation de nature différente permet de réduire de manière significative l'activation par le patient d'une commande non-volontaire.

Selon un mode de réalisation de l'invention, le système de commande est agencé pour activer une commande du dispositif médical lors du déclenchement simultané du premier et du deuxième élément d'activation, alors que selon un autre mode le système de commande est agencé pour activer une commande du dispositif médical lors du déclenchement successif du premier et du deuxième élément d'activation. De préférence le déclenchement successif du premier et du deuxième élément d'activation est effectué dans un intervalle de temps inférieur à 1 seconde, de préférence inférieur à 500 millisecondes, encore de préférence inférieur ou égal à 100 millisecondes.

Selon un mode de réalisation particulier de l'invention, le premier et le deuxième élément d'activation sont disposés à des emplacements différents du système de commande, de préférence sur des faces opposées du système de commande alors que selon un autre mode de réalisation le premier et le deuxième élément d'activation sont disposés à un même emplacement du système de commande de sorte à se superposer.

Selon un mode de réalisation de l'invention, le premier élément d'activation comprend un capteur sélectionné dans un groupe consistant en un capteur tactile, résistif, capacitif, à induction, à infrarouge, optique, magnétique ou à reconnaissance d'empreinte digitale. De préférence, le capteur à reconnaissance d'empreintes digitales est configuré pour reconnaitre au moins une première succession d'empreintes digitales pour activer une première commande du dispositif médical et au moins une deuxième succession d'empreintes digitales pour activer une deuxième commande du dispositif médical, la première commande étant différente de la deuxième.

Selon un mode de réalisation de l'invention, l'élément d'activation rapporté est distinct du système de commande et constitué par une partie d'un corps humain ou animal, de préférence un doigt, un stylet ou un badge ou une puce notamment magnétique ou électro-magnétique.

Selon un autre mode de réalisation de l'invention, le premier élément d'activation est agencé pour être déclenché par proximité avec l'élément d'activation rapporté à une distance inférieure à 5 millimètres, de préférence inférieure à 1 millimètre. Alternativement, le premier élément d'activation est agencé pour être déclenché par contact avec l'élément d'activation rapporté.

Dans un mode de réalisation particulier de l'invention, le deuxième élément d'activation comprend un capteur de changement d'état qui mesure un changement d'état sous l'effet d'une pression exercée sur ledit deuxième élément d'activation qui entraine un déplacement d'au moins une partie dudit deuxième élément d'activation. Le deuxième élément d'activation est de préférence sélectionné dans un groupe consistant en un bouton poussoir, un interrupteur ou un commutateur électrique.

Selon un autre mode de réalisation, le déclenchement dudit deuxième élément d'activation est agencé pour mettre sous tension le système de commande ce qui permet d'éviter de le maintenir dans une veille prolongée consommatrice d'énergie.

De préférence le système de commande est configuré pour activer une commande du dispositif médical à distance. C'est-à-dire que le système de commande est situé hors du corps humain ou animal et sans contact avec ce dernier. On comprend donc que le système de commande n'est pas destiné à être implanté et n'est pas implanté dans le corps humain ou animal. Il est ainsi prévu que le système de commande comprenne au moins un émetteur radiofréquence configuré pour communiquer sans fil avec le dispositif médical, l'émetteur radiofréquence étant configuré pour être appairé avec un récepteur radiofréquence dudit dispositif médical préalablement à l'activation d'une commande du dispositif médical par le système de commande.

Selon un mode de réalisation particulier de l'invention, le système de commande comprend un afficheur indiquant l'activation d'une commande du dispositif médical par le système de commande.

De préférence, le système de commande est configuré pour commander un dispositif médical d'occlusion d'un conduit naturel du corps humain ou animal, le dispositif médical étant sélectionné dans un groupe consistant en un sphincter artificiel, un muscle artificiel, un stimulateur électrique, un anneau gastrique, un neurostimulateur ou un implant pénien.

L'invention s'étend à un équipement médical comprenant un système de commande comme défini ci-dessus et un dispositif médical agencé pour être implanté dans un corps humain ou animal.

L'invention s'étend en outre à une méthode de commande à distance d'un dispositif médical agencé pour être implanté dans un corps humain ou animal, ladite méthode comprenant au moins les étapes consistant à :
- fournir un système de commande comprenant au moins un premier et un deuxième élément d'activation pour commander à distance ledit dispositif médical, ledit premier élément d'activation étant déclenché par proximité avec un élément d'activation rapporté, ledit deuxième élément d'activation étant déclenché par une pression exercée sur ledit deuxième élément d'activation,
- activer une commande du dispositif médical lors du déclenchement du premier et du deuxième élément d'activation du le système de commande.

Dans un mode de réalisation de la méthode selon l'invention, le système de commande active une commande du dispositif médical lors du déclenchement simultané du premier et du deuxième élément d'activation, alors que selon un autre mode le système de commande active une commande du dispositif médical lors du déclenchement successif du premier et du deuxième élément d'activation. De préférence, le déclenchement successif du premier et du deuxième élément d'activation est effectué dans un intervalle de temps inférieur à 1 seconde, de préférence inférieur à 500 millisecondes, encore de préférence inférieur ou égal à 100 millisecondes.

Selon un mode de réalisation particulier de la méthode, on dispose le premier et le deuxième élément d'activation à des emplacements différents du système de commande, de préférence sur des faces opposées du système de commande, alors que selon un autre mode de réalisation on dispose le premier et le deuxième élément d'activation à un même emplacement du système de commande.

Selon un autre mode de réalisation de la méthode, on équipe ledit premier élément d'activation d'un capteur sélectionné dans un groupe consistant en un capteur tactile, résistif, capacitif, à induction, à infrarouge, optique, magnétique ou à reconnaissance d'empreinte digitale. De préférence, le capteur à reconnaissance d'empreintes digitales reconnait au moins une première succession d'empreintes digitales pour activer une première commande du dispositif médical et au moins une deuxième succession d'empreintes digitales pour activer une deuxième commande du dispositif médical, la première commande étant différente de la deuxième.

Selon encore un autre mode de réalisation de la méthode, on fournit ledit élément d'activation rapporté distinct du système de commande et constitué par une partie d'un corps humain ou animal, de préférence un doigt, ou par un stylet ou par un badge ou une puce notamment magnétique ou électro-magnétique.

Selon un mode de réalisation de la méthode, on déclenche ledit premier élément d'activation par proximité avec l'élément d'activation rapporté à une distance inférieure à 5 mm, de préférence inférieure à 1mm. Alternativement, on déclenche ledit premier élément d'activation par contact avec l'élément d'activation rapporté.

Dans un mode de réalisation particulier de la méthode, on équipe ledit deuxième élément d'activation d'un capteur de changement d'état qui mesure un changement d'état sous l'effet d'une pression exercée sur ledit deuxième élément d'activation entrainant un déplacement d'au moins une partie dudit deuxième élément d'activation.

Le deuxième élément d'activation est de préférence sélectionné dans un groupe consistant en un bouton poussoir, un interrupteur ou un commutateur électrique.

Dans un autre mode de réalisation particulier de la méthode, on déclenche le deuxième élément d'activation pour mettre sous tension le système de commande.

De préférence le système de commande est configuré pour activer une commande du dispositif médical à distance. Le système de commande joue ainsi le rôle d'une télécommande. En particulier, on équipe le système de commande d'au moins un émetteur radiofréquence communiquant sans fil avec le dispositif médical et on appaire l'émetteur radiofréquence du système de commande avec un récepteur radiofréquence du dispositif médical préalablement à l'activation d'une commande du dispositif médical par le système de commande.

Selon un autre mode de réalisation particulier de la méthode, on affiche l'activation d'une commande du dispositif médical par le système de commande au moyen d'un afficheur disposé sur le système de commande.

De préférence, on commande un dispositif médical d'occlusion d'un conduit naturel du corps humain ou animal sélectionné dans un groupe consistant en un sphincter artificiel, un muscle artificiel, un stimulateur électrique, un anneau gastrique, un neurostimulateur ou un implant pénien.

### BREVE DESCRIPTION DES FIGURES

D'autres caractéristiques et avantages de l'invention ressortiront de la description détaillée qui va suivre, en référence aux dessins annexés sur lesquels :
- la figure 1 illustre une vue schématique du système de commande selon un mode de réalisation de l'invention et du dispositif médical implanté dans un corps humain ou animal ;
- la figure 2 illustre une vue de l'architecture du système de commande selon l'invention ;
- la figure 3 illustre schématiquement l'activation d'une commande du dispositif médical par le système de commande.

### DESCRIPTION DETAILLEE DE MODES DE REALISATION

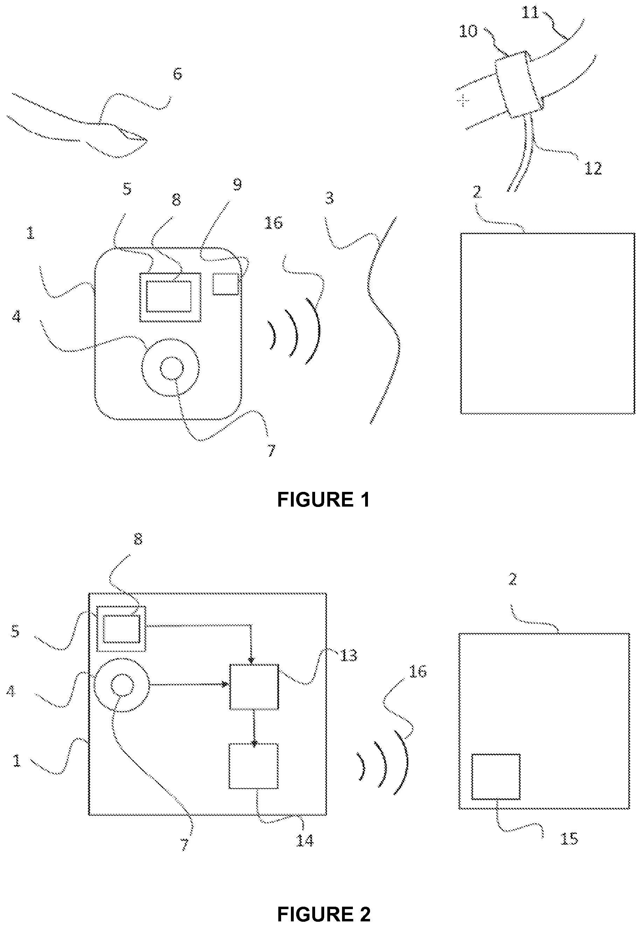

La figure 1 illustre de manière schématique un système de commande 1 à distance d'un dispositif médical 2. Le dispositif médical 2 est montré implanté dans le corps humain ou animal du patient qui est représenté en partie par la référence 3 sur la figure 1 symbolisant la peau du corps. Sur la figure 1, on note que ce qui est représenté à droite de la peau 3 du corps humain ou animal désigne ce qui est implanté dans le corps, dans le cas présent le dispositif médical 2 qui a par exemple été implanté au cours d'une opération chirurgicale. A gauche de la peau 2 du corps humain ou animal se trouve donc ce qui n'est pas implanté dans le corps, dans le cas d'espèce, le système de commande 1.

Selon l'invention, le système de commande 1 comprend un premier 4 et un deuxième 5 élément d'activation pour commander le dispositif médical 2. Le premier élément d'activation 4 est agencé pour être déclenché par proximité avec un élément rapporté d'activation 6, alors que le deuxième élément d'activation 5 est agencé pour être déclenché par une pression exercée sur ledit deuxième élément d'activation. Les deux éléments d'activation sont donc de nature différente, c'est-à-dire qu'ils sont déclenchés par des technologies différentes qui n'ont pas d'interaction l'une avec l'autre, et cela a pour effet de réduire significativement les activations de commandes involontaires par le patient, comme cela est détaillé ci-après.

En particulier, le premier élément d'activation 4 comprend un capteur de proximité ou de contact 7 sélectionné dans un groupe consistant en un capteur tactile, résistif, capacitif, à induction, à infrarouge, optique, magnétique ou à reconnaissance d'empreinte digitale. Par « proximité » on entend que le capteur 7 détecte la présente de l'élément rapporté d'activation 6 lorsque celui-ci se trouve à une distance inférieure à 5 millimètres du capteur 7, de préférence inférieure à 1 millimètre.

Ce type de capteur 7 mesure ou détecte la présence d'un élément d'activation rapporté 6 lorsqu'il se situe à proximité ou en contact physique avec le capteur ce qui a pour effet de déclencher le premier élément d'activation. Selon l'invention, l'élément d'activation rapporté 6 est distinct du système de commande 1, c'est-à-dire qu'il n'en fait pas partie. De préférence, il est constitué par une partie d'un corps humain ou animal, par exemple un doigt 6 mais il peut également se présenter sous la forme d'un stylet ou un badge notamment un badge ou puce magnétique ou électro-magnétique.

Dans le cas d'un capteur 7 à reconnaissance d'empreintes digitales il est prévu que le capteur soit configuré pour reconnaitre au moins une première succession d'empreintes digitales de sorte à activer une première commande, par exemple une ouverture de l'urètre et au moins une deuxième succession d'empreintes digitales différente de la première pour activer une deuxième commande, par exemple une demande d'information sur la pression exercée par le dispositif médical sur le conduit naturel ou sur l'état ouvert ou obturé du conduit.

Le deuxième élément d'activation 5 comprend quant à lui un capteur 8 qui mesure un changement d'état. En particulier, une pression exercée sur le deuxième élément d'activation 5 entraine un déplacement d'au moins une partie dudit deuxième élément d'activation et donc un changement d'état par exemple entre une position haute et une position basse d'une partie du deuxième élément d'activation telle que mesurée ou détectée par le capteur 8.

Par exemple, ce deuxième élément d'activation 5 est sélectionné dans un groupe consistant en un bouton poussoir, un interrupteur ou un commutateur électrique. Le bouton poussoir est équipé d'un ressort de rappel qui permet au bouton de revenir à sa position initiale lorsque plus aucune pression n'est exercée dessus.

De préférence, le premier 4 et le deuxième 5 élément d'activation sont disposés dans un logement en creux de sorte à éviter l'activation d'une commande involontaire qui serait déclenchée par un élément rapporté passant à proximité de la surface du système de commande1. En effet dans ce cas, il serait nécessaire que ledit élément rapporté pénètre dans le creux pour activer une commande, un simple contact avec la surface ne suffirait pas.

Dans le mode de réalisation illustré sur la figure 1, le premier 4 et le deuxième 5 élément d'activation sont disposés à des emplacements différents du système de commande. Sur l'exemple la figure 1, le système de commande 1 est de forme sensiblement parallélépipédique de sorte que le premier 4 et le deuxième 5 élément d'activation soient disposés sur une même face du système de commande 1, juxtaposés l'un à l'autre.

Alternativement, le premier 4 et le deuxième 5 élément d'activation sont disposés sur des faces opposées du système de commande 1. Avantageusement, ce mode de réalisation permet à l'utilisateur d'activer une commande du dispositif médical 2 implanté par contact d'un doigt, l'index par exemple, avec le premier élément d'activation 4 et par pression d'un autre doigt, par exemple le pouce ou le majeur, sur le deuxième élément d'activation 5. Ce mode de réalisation présente une ergonomie particulière adaptée aux déclenchements des premier et deuxième éléments d'activation par les doigts d'une main puisque, dans ce cas, le dispositif de commande 1 est maintenu aisément et fermement entre le pouce et l'index ou le majeur.

Encore alternativement mais de manière non-illustrée, le premier 4 et le deuxième 5 élément d'activation sont disposés à un même emplacement du système de commande 1 de sorte qu'ils se superposent et ne font ainsi apparaître, pour l'utilisateur, qu'un seul élément d'activation. Cette configuration présente l'avantage de permettre le déclenchement des premier et deuxième éléments d'activation et donc l'activation d'une commande du dispositif médical au moyen d'une seule action du patient.

Le système de commande 1 comprend en outre un afficheur 9 qui indique, par exemple au moyen d'un signal lumineux, que l'activation d'une commande par le système de commande 1 a bien été prise en compte et que, par exemple, la commande consistant en une ouverture d'un conduit anatomique sera effectuée par le dispositif médical 2.

A cet effet et dans l'exemple de réalisation particulier du sphincter urinaire artificiel, il est prévu que le dispositif médical 2 comprenne au moins une manchette occlusive gonflable 10 entourant le conduit naturel 11, l'urètre dans ce cas, et une connexion fluidique tubulaire 12 dont une extrémité est connectée à la manchette 10 pour créer une liaison fluidique avec le reste du dispositif médical 2.

En particulier le dispositif médical 2 inclut également une unité de contrôle ainsi qu'une pompe (non-illustrées pour des raisons de clarté), la pompe incluant elle-même un réservoir à volume variable. Le réservoir de la pompe est rempli de fluide et connectée à l'autre extrémité de la connexion fluidique tubulaire 12. Ainsi le réservoir de la pompe, la connexion fluidique tubulaire 12 et la manchette occlusive 10 forment un même circuit fluidique, le fluide pouvant être transféré de manière bidirectionnelle du réservoir à la pompe.

En fonctionnement, l'unité contrôle la pompe de sorte à augmenter ou réduire le volume de fluide dans le réservoir et donc la quantité de fluide dans la manchette 10 via le circuit fluidique. Cet agencement a pour effet de permettre de gonfler ou dégonfler la manchette, d'augmenter ou de diminuer la compression exercée et donc d'obturer par compression ou de libérer l'urètre 11 évitant ainsi les fuites urinaires des patients souffrant d'incontinence.

Le dispositif médical 2 incluant la manchette occlusive 10, la connexion fluidique tubulaire 12, l'unité de commande, la pompe et le réservoir sont donc implantés dans le corps humain ou animal (à droite de la peau 3 sur la figure 1) pour être commandés à distance par le système de commande 1 situé à l'extérieur du corps (à gauche de la peau 3 sur la figure 1).

Par ailleurs, il est envisagé selon l'invention que la commande du dispositif médical 2 puisse consister en une activation ou une désactivation du dispositif, par exemple pour mettre en oeuvre un mode de fonctionnement particulier du dispositif, de configurer ses paramètres internes ou encore de requérir des informations de la part du dispositif, par exemple de sorte à connaître l'état du dispositif, en particulier l'état de charge de la source d'énergie du dispositif.

Sur la figure 2, on montre le détail de l'architecture du système de commande 1 et son fonctionnement général en liaison avec le dispositif médical 2.

En particulier, le système de commande 1 comprend un capteur de proximité 7 associé au premier élément d'activation 4, un capteur de changement d'état 8 associé au deuxième élément d'activation 5, une unité de contrôle/commande 13 et un émetteur/récepteur 14 de signaux radiofréquences 16.

L'unité de contrôle/commande 13 est connectée au premier 4 et deuxième 5 élément d'activation ainsi qu'à l'émetteur/récepteur 14. Elle est donc apte à recevoir du premier élément d'activation 4 un signal de déclenchement indiquant qu'un élément d'activation rapporté 6, par exemple le doigt d'une main, a été détecté à proximité ou en contact avec le capteur de proximité 7. De même, l'unité de contrôle/commande 13 est apte à recevoir un signal de déclenchement en provenance du deuxième élément d'activation 5 indiquant qu'une pression a été exercée sur le capteur de changement d'état 8.

A la réception des signaux de déclenchement des premier 4 et deuxième 5 élément d'activation, l'unité de contrôle/commande 13 traite les signaux de déclenchement et détermine un signal d'activation d'une commande à destination du dispositif médical 2 qui est transmis à l'émetteur/récepteur 14.

L'émetteur/récepteur 14 recevant le signal d'activation de la commande émet un signal radiofréquence 16 à destination de l'émetteur/récepteur 15 du dispositif médical 2. Ce signal est ensuite envoyé par l'émetteur/récepteur 15 à l'unité de contrôle (non-illustrée) du dispositif médical 2 pour être traité et commander l'ouverture de l'urètre 11 dans l'exemple de réalisation d'un sphincter urinaire artificiel comme expliqué ci-dessus.

Préalablement à l'activation d'une commande du dispositif médical 2 par le système de commande 1 et préalablement à la communication radiofréquence entre le système de commande 1 et le dispositif médical 2, il est, de préférence, prévu un appairage de l'émetteur/récepteur 14 du système de commande avec l'émetteur/récepteur 15 du dispositif médical 2.

Selon un mode de réalisation particulier de l'appairage entre les émetteurs/récepteurs 14 et 15, le système de commande 1, équipé d'un lecteur de code à barres, lit le code d'appairage du dispositif médical 2 disposé par exemple sur l'emballage du dispositif médical sous la forme d'un code à barres. Ce code d'appairage constitue une clef secrète qui autorise la communication entre les deux émetteurs/récepteurs.

Cette étape d'appairage permet d'établir une communication sécurisée mais aussi de s'assurer que le patient commande et communique avec le bon dispositif médical. En effet, compte tenu de la distance de portée des émetteurs-récepteurs de radiofréquences qui est typiquement de quelques mètres, si plusieurs dispositifs médicaux se trouvent à proximité du système de commande, sans étape d'appairage, il existerait un risque de confusion entraînant la communication avec un dispositif médical différent de celui avec lequel on souhaite communiquer.

A l'issue de l'étape d'appairage, le système de commande 1 et le dispositif médical 2 sont configurés pour communiquer ensemble de manière sécurisée et le système de commande 1 est apte à activer une commande du dispositif médical 2.

De préférence, la bande de fréquences pour la communication entre les deux émetteurs/récepteurs 14 et 15 est une bande de fréquences dédiée et réservée aux dispositifs médicaux, distincte de la bande de fréquences destinée aux applications grand public, ce qui permet d'assurer la sécurité des communications entre le système de commande et le dispositif médical implanté et d'éviter l'activation d'une commande qui ne proviendrait pas du système de commande 1 du patient.

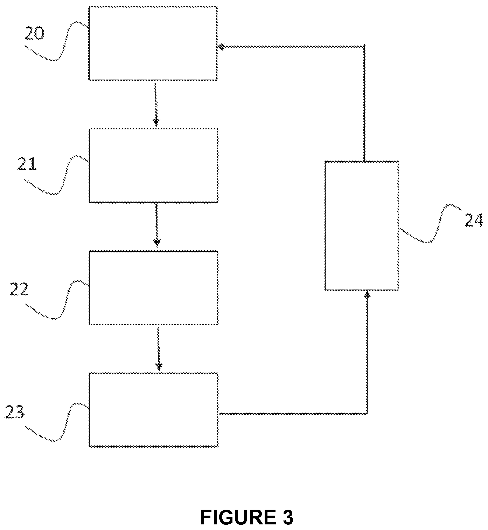

En lien avec la figure 3, nous allons maintenant détailler un exemple de réalisation de la méthode d'activation d'une commande du dispositif médical.

Selon cet exemple particulier de réalisation, dans lequel le système de commande est dans un état initial hors tension, le patient déclenche à l'étape 20 le deuxième élément d'activation 5, par exemple un bouton poussoir, en exerçant une pression dessus avec un doigt ce qui a pour effet selon l'invention de mettre le système de commande 1 sous tension à l'étape 21.

Avantageusement, cette étape de mise sous-tension permet d'économiser de l'énergie lorsque le système est hors-tension.

Une fois sous tension et dans une configuration selon laquelle le premier 4 et le deuxième 5 élément d'activation sont positionnés à un même emplacement sur le dispositif de commande 1, le doigt toujours positionné sur le deuxième élément d'activation 5 déclenche le premier élément d'activation 4 à l'étape 22.

Le déclenchement successif du premier 4 et deuxième 5 élément d'activation, effectué de préférence dans un intervalle de temps inférieur à 100 millisecondes, entraîne l'activation d'une commande du dispositif médical 2 à l'étape 23.

Le système de commande 1 communique avec le dispositif médical 2 comme décrit ci-dessus et active une commande consistant, par exemple, à exercer une diminution de la pression de la manchette 10 sur le conduit naturel 11.

Il est prévu que la prise en compte d'une commande et son exécution par le dispositif médical 2 soit indiqué sur le système de commande 1 au moyen d'un afficheur avec un affichage lumineux, par exemple du type diode électro-luminescente LED.

À la suite de l'activation de la commande à l'étape 23, on démarre à l'étape 24 une horloge du système de commande 1 qui compte le temps avant de mettre le système 1 à nouveau hors tension.

Alternativement, il est envisagé que le système de commande 1 soit sous-tension de manière permanente. Dans ce cas et dans une configuration selon laquelle les éléments d'activation 4 et 5 sont disposés à des emplacements différents sur le système 1, alors le déclenchement simultané des éléments d'activation 4 et 5 entrainera l'activation d'une commande.

Pour économiser son énergie, le dispositif médical peut être mis automatiquement hors-tension en-dehors des phases d'activation. La communication avec le système de commande nécessite donc un réveil (mise sous tension) préalable du dispositif médical. Un tel réveil peut être mis en oeuvre par une tentative de communication continue (en boucle) par le système de commande une fois que celui-ci a été activé par déclenchement des deux éléments d'activation. Le réveil du dispositif médical peut être indiqué sur le système de commande par l'afficheur mentionné plus haut, par exemple par l'allumage d'une LED. Pour mettre en oeuvre une telle fonction de réveil, le système de commande peut comprendre deux antennes avec des polarisations différentes adaptées pour mettre sous tension le dispositif médical. Grâce à ces deux polarisations, la communication entre le système de commande et le dispositif médical est améliorée pour différentes positions et/ou orientations relatives du système de commande et du dispositif médical. Tant que la communication n'est pas établie entre le système de commande et le dispositif médical, l'utilisateur manipule le système de commande selon différentes positions et/ou orientations jusqu'à ce que l'afficheur du système de commande lui indique que le dispositif médical a été réveillé. Un avantage de cette configuration du système de commande est que le réveil du dispositif médical ne nécessite qu'un seul déclenchement des éléments d'activation du système de commande, et que la durée d'appairage est ainsi réduite.

## Revendications

1. Système de commande (1) à distance d'un dispositif médical (2) agencé pour être implanté dans un corps humain ou animal, ledit système de commande comprenant au moins un premier (4) et un deuxième (5) élément d'activation pour commander ledit dispositif médical, ledit premier élément d'activation étant agencé pour être déclenché par proximité avec un élément d'activation rapporté (6), ledit deuxième élément d'activation étant agencé pour être déclenché par une pression exercée sur ledit deuxième élément d'activation, le système de commande étant configuré pour établir une communication avec le dispositif médical dans une bande de fréquences dédiée aux dispositifs médicaux et pour activer une commande du dispositif médical lors du déclenchement du premier et du deuxième élément d'activation simultanément ou successivement dans un intervalle de temps inférieur à 1 seconde, de préférence inférieur à 500 millisecondes, encore de préférence inférieur ou égal à 100 millisecondes.

2. Système de commande selon la revendication 1, dans lequel le premier (4) et le deuxième (5) élément d'activation sont disposés à des emplacements différents du système de commande (1), de préférence sur des faces opposées du système de commande (1).

3. Système de commande selon la revendication 1 dans lequel le premier (4) et le deuxième (5) élément d'activation sont disposés à un même emplacement du système de commande (1) de sorte à se superposer.

4. Système de commande selon l'une des revendications 1 à 3, dans lequel ledit premier élément d'activation (4) comprend un capteur (7) sélectionné dans un groupe consistant en un capteur tactile, résistif, capacitif, à induction, à infrarouge, optique, magnétique ou à reconnaissance d'empreinte digitale.

5. Système de commande selon l'une des revendications 1 à 4, dans lequel ledit premier élément d'activation comprend un capteur (7) à reconnaissance d'empreintes digitales configuré pour reconnaitre au moins une première succession d'empreintes digitales pour activer une première commande du dispositif médical et au moins une deuxième succession d'empreintes digitales pour activer une deuxième commande du dispositif médical, la première commande étant différente de la deuxième.

6. Système de commande selon l'une des revendications 1 à 5, dans lequel ledit premier élément d'activation est agencé pour être déclenché par proximité avec un élément d'activation rapporté (6) distinct du système de commande (1) et choisi parmi une partie d'un corps humain ou animal, de préférence un doigt, un stylet ou un badge ou puce notamment magnétique ou électro-magnétique.

7. Système de commande selon l'une des revendications 1 à 6, dans lequel ledit premier élément d'activation (4) est agencé pour être déclenché par proximité avec l'élément d'activation rapporté (6) à une distance inférieure à 5 millimètres, de préférence inférieure à 1 millimètre.

8. Système de commande selon l'une des revendications 1 à 5, dans lequel ledit premier élément d'activation (4) est agencé pour être déclenché par contact avec l'élément d'activation rapporté (6).

9. Système de commande selon l'une des revendications 1 à 8, dans lequel ledit deuxième élément d'activation (5) comprend un capteur de changement d'état (8) qui mesure un changement d'état sous l'effet d'une pression exercée sur ledit deuxième élément d'activation (5) qui entraine un déplacement d'au moins une partie dudit deuxième élément d'activation.

10. Système de commande selon l'une des revendications 1 à 9, dans lequel ledit deuxième élément d'activation (5) est sélectionné dans un groupe consistant en un bouton poussoir, un interrupteur et un commutateur électrique.

11. Système de commande selon l'une des revendications 1 à 10, dans lequel un déclenchement (20) dudit deuxième élément d'activation (5) est agencé pour mettre sous tension (21) le système de commande (1).

12. Système de commande selon l'une des revendications 1 à 11, dans lequel ledit système de commande (1) est configuré pour activer une commande (23) du dispositif médical à distance, hors du corps humain ou animal et sans contact avec le corps humain ou animal.

13. Système de commande selon l'une des revendications 1 à 12, dans lequel ledit système de commande (1) comprend au moins un émetteur radiofréquence (14) configuré pour communiquer sans fil avec le dispositif médical.

14. Système de commande selon la revendication 13, dans lequel ledit émetteur radiofréquence (14) dudit système de commande est configuré pour être appairé avec un récepteur radiofréquence (15) dudit dispositif médical (2) préalablement à l'activation d'une commande du dispositif médical par le système de commande.

15. Système de commande selon l'une des revendications 1 à 14, dans lequel ledit système de commande comprend un afficheur (9) indiquant l'activation d'une commande du dispositif médical (2) par le système de commande (1).

16. Système de commande selon l'une des revendications 1 à 15, dans lequel le système de commande (1) est configuré pour commander un dispositif médical (2) d'occlusion d'un conduit naturel (11) du corps humain ou animal.

17. Système de commande selon l'une des revendications 1 à 16, dans lequel le système de commande (1) est configuré pour commander un dispositif médical agencé pour être implanté dans un corps humain ou animal et sélectionné dans un groupe consistant en un sphincter artificiel, un muscle artificiel, un stimulateur électrique, un anneau gastrique, un neurostimulateur ou un implant pénien.

18. Equipement médical comprenant un système de commande (1) selon l'une des revendications précédentes et un dispositif médical (2) agencé pour être implanté dans un corps humain ou animal.

## Patentansprüche

1. Fernsteuerungssystem (1) einer medizinischen Vorrichtung (2), die zur Implantation in einen menschlichen oder tierischen Körper eingerichtet ist, wobei das Steuerungssystem mindestens ein erstes (4) und ein zweites (5) Aktivierungselement zur Steuerung der medizinischen Vorrichtung umfasst, wobei das erste Aktivierungselement eingerichtet ist, durch Nähe zu einem angebrachten Aktivierungselement (6) ausgelöst zu werden, wobei das zweite Aktivierungselement eingerichtet ist, durch einen auf das zweite Aktivierungselement ausgeübten Druck ausgelöst zu werden, wobei das Steuerungssystem ausgelegt ist, um eine Kommunikation mit der medizinischen Vorrichtung in einem für medizinische Vorrichtungen bestimmten Frequenzband herzustellen und um eine Steuerung der medizinischen Vorrichtung bei Auslösung des ersten und des zweiten Aktivierungselements gleichzeitig oder nacheinander innerhalb eines Zeitintervalls von weniger als 1 Sekunde, vorzugsweise weniger als 500 Millisekunden, noch bevorzugter weniger als oder gleich 100 Millisekunden, zu aktivieren.

2. Steuerungssystem nach Anspruch 1, wobei das erste (4) und das zweite (5) Aktivierungselement an verschiedenen Stellen des Steuerungssystems (1), vorzugsweise auf gegenüberliegenden Seiten des Steuerungssystems (1), angeordnet sind.

3. Steuerungssystem nach Anspruch 1, wobei das erste (4) und das zweite (5) Aktivierungselement an derselben Stelle des Steuerungssystems (1) derart angeordnet sind, dass sie einander überlagern.

4. Steuerungssystem nach einem der Ansprüche 1 bis 3, wobei das erste Aktivierungselement (4) einen Sensor (7) umfasst, der aus einer Gruppe ausgewählt ist, die aus einem taktilen, resistiven, kapazitiven, induktiven, Infrarot-, optischen, magnetischen oder Sensor mit Fingerabdruckerkennung besteht.

5. Steuerungssystem nach einem der Ansprüche 1 bis 4, wobei das erste Aktivierungselement einen Sensor (7) mit Fingerabdruckerkennung umfasst, der ausgelegt ist, um mindestens eine erste Abfolge Fingerabdrücke zur Aktivierung einer ersten Steuerung der medizinischen Vorrichtung und mindestens eine zweite Abfolge von Fingerabdrücken zur Aktivierung einer zweiten Steuerung der medizinischen Vorrichtung zu erkennen, wobei sich die erste Steuerung von der zweiten Steuerung unterscheidet.

6. Steuerungssystem nach einem der Ansprüche 1 bis 5, wobei das erste Aktivierungselement eingerichtet ist, um durch Nähe zu einem angebrachten Aktivierungselement (6) ausgelöst zu werden, das vom Steuerungssystem (1) verschieden und aus einem Teil eines menschlichen oder tierischen Körpers, vorzugsweise einem Finger, einem Stift oder einem insbesondere magnetischen oder elektromagnetischen Badge oder Chip ausgewählt ist.

7. Steuerungssystem nach einem der Ansprüche 1 bis 6, wobei das erste Aktivierungselement (4) eingerichtet ist, um durch Nähe zu dem angebrachten Aktivierungselement (6) in einem Abstand von weniger als 5 Millimeter, vorzugsweise weniger als 1 Millimeter, ausgelöst zu werden.

8. Steuerungssystem nach einem der Ansprüche 1 bis 5, wobei das erste Aktivierungselement (4) eingerichtet ist, um durch Kontakt mit dem angebrachten Aktivierungselement (6) ausgelöst zu werden.

9. Steuerungssystem nach einem der Ansprüche 1 bis 8, wobei das zweite Aktivierungselement (5) einen Zustandsänderungssensor (8) umfasst, der eine Zustandsänderung unter der Wirkung eines Drucks misst, der auf das zweite Aktivierungselement (5) ausgeübt wird, der eine Bewegung von mindestens einem Teil des zweiten Aktivierungselements bewirkt.

10. Steuerungssystem nach einem der Ansprüche 1 bis 9, wobei das zweite Aktivierungselement (5) aus einer Gruppe ausgewählt ist, die aus einem Druckknopf, einem Schalter und einem elektrischen Umschalter besteht.

11. Steuerungssystem nach einem der Ansprüche 1 bis 10, wobei eine Auslösung (20) des zweiten Aktivierungselements (5) eingerichtet ist, um das Steuerungssystem (1) unter Spannung zu setzen (21).

12. Steuerungssystem nach einem der Ansprüche 1 bis 11, wobei das Steuerungssystem (1) ausgelegt ist, um eine Steuerung (23) der medizinischen Vorrichtung aus der Ferne, außerhalb des menschlichen oder tierischen Körpers und ohne Kontakt mit dem menschlichen oder tierischen Körper, zu aktivieren.

13. Steuerungssystem nach einem der Ansprüche 1 bis 12, wobei das Steuerungssystem (1) mindestens einen Hochfrequenzsender (14) umfasst, der ausgelegt ist, um drahtlos mit der medizinischen Vorrichtung zu kommunizieren.

14. Steuerungssystem nach Anspruch 13, wobei der Hochfrequenzsender (14) des Steuerungssystems ausgelegt ist, um mit einem Hochfrequenzempfänger (15) der medizinischen Vorrichtung (2) vor der Aktivierung einer Steuerung der medizinischen Vorrichtung durch das Steuerungssystem gepairt zu werden.

15. Steuerungssystem nach einem der Ansprüche 1 bis 14, wobei das Steuerungssystem ein Display (9) umfasst, das die Aktivierung einer Steuerung der medizinischen Vorrichtung (2) durch das Steuerungssystem (1) anzeigt.

16. Steuerungssystem nach einem der Ansprüche 1 bis 15, wobei das Steuerungssystem (1) ausgelegt ist, um eine medizinische Vorrichtung (2) zum Verschließen eines natürlichen Kanals (11) des menschlichen oder tierischen Körpers zu steuern.

17. Steuerungssystem nach einem der Ansprüche 1 bis 16, wobei das Steuerungssystem (1) ausgelegt ist, um eine medizinische Vorrichtung zu steuern, die zur Implantation in einen menschlichen oder tierischen Körper eingerichtet und aus einer Gruppe ausgewählt ist, die aus einem künstlichen Schließmuskel, einem künstlichen Muskel, einem elektrischen Schrittmacher, einem Magenband, einem Neurostimulator oder einem Penisimplantat besteht.

18. Medizinische Ausrüstung, die ein Steuerungssystem (1) nach einem der vorhergehenden Ansprüche und eine medizinische Vorrichtung (2), die zur Implantation in einen menschlichen oder tierischen Körper eingerichtet ist, umfasst.

## Claims

1. A system (1) for remotely commanding a medical device (2) arranged to be implanted in a human or animal body, said command system comprising at least a first (4) and a second (5) activation element for commanding said medical device, said first activation element being arranged to be triggered by proximity to an added activation element (6), said second activation element being arranged to be triggered by pressure exerted on said second activation element, the command system being configured to establish a communication with the medical device in a frequency band dedicated to medical devices and to activate a command of the medical device upon triggering of the first and second activation elements simultaneously or successively in a time interval of less than 1 second, preferably less than 500 milliseconds, still preferably less than or equal to 100 milliseconds.

2. The command system according to claim 1, wherein the first (4) and second (5) activation elements are disposed at different locations of the command system (1), preferably on opposite sides of the command system (1).

3. The command system according to claim 1 wherein the first (4) and second (5) activation elements are disposed at the same location of the command system (1) so as to overlap.

4. The command system according to one of the preceding claims 1 to 3, wherein said first activation element (4) comprises a sensor (7) selected from a group consisting of a tactile, resistive, capacitive, induction, infrared, optical, magnetic or fingerprint recognition sensor.

5. The command system according to one of claims 1 to 4, wherein said first activation element comprises a fingerprint recognition sensor (7) configured to recognise at least a first succession of fingerprints to activate a first command of the medical device and at least a second succession of fingerprints to activate a second command of the medical device, the first command being different from the second one.

6. The command system according to one of claims 1 to 5, wherein said first activation element is arranged to be triggered by proximity to an added activation element (6) distinct from the command system (1) and chosen from a part of a human or animal body, preferably a finger, a stylus or a badge or chip, in particular magnetic or electro-magnetic.

7. The command system according to one of claims 1 to 6, wherein said first activation element (4) is arranged to be triggered by proximity to the added activation element (6) at a distance of less than 5 millimetres, preferably less than 1 millimetre.

8. The command system according to one of claims 1 to 6, wherein said first activation element (4) is arranged to be triggered by contact with the added activation element (6).

9. The command system according to one of claims 1 to 8, wherein said second activation element (5) comprises a change of state sensor (8) which measures a change of state under the effect of pressure exerted on said second activation element (5) which causes a movement of at least a part of said second activation element.

10. The command system according to one of claims 1 to 9, wherein said second activation element (5) is selected from a group consisting of a push button, a switch and an electrical commutator.

11. The command system according to one of claims 1 to 10, wherein triggering (20) of said second activation element (5) is arranged to switch on (21) the command system (1).

12. The command system according to one of claims 1 to 11, wherein said command system (1) is configured to activate a command (23) of the medical device remotely, outside the human or animal body and without contact with the human or animal body.

13. The command system according to one of claims 1 to 12, wherein said command system (1) comprises at least one radio frequency transmitter (14) configured to communicate wirelessly with the medical device.

14. The command system according to claim 13, wherein said radio frequency transmitter (14) of said command system is configured to be paired with a radio frequency receiver (15) of said medical device (2) prior to activation of a command of the medical device by the command system.

15. The command system according to one of claims 1 to 14, wherein said command system comprises a display (9) indicating activation of a command of the medical device (2) by the command system (1).

16. The command system according to one of claims 1 to 15, wherein the command system (1) is configured to command a medical device (2) for occluding a natural duct (11) of the human or animal body.

17. The command system according to one of claims 1 to 16, wherein the command system (1) is configured to command a medical device arranged to be implanted in a human or animal body and selected from a group consisting of an artificial sphincter, an artificial muscle, an electrical stimulator, a gastric ring, a neurostimulator or a penile implant.

18. A piece of medical equipment comprising a command system (1) according to one of the preceding claims and a medical device (2) arranged to be implanted in a human or animal body.
